Europäisches Patentamt

European Patent Office

Office européen des brevets

(19)

(11) Veröffentlichungsnummer: **0 181 433**
A1

(12) # EUROPÄISCHE PATENTANMELDUNG

(21) Anmeldenummer: 85107410.4

(22) Anmeldetag: 15.06.85

(51) Int. Cl.⁴: **A 61 B 17/58**

(30) Priorität: 27.10.84 DE 8431616 U

(43) Veröffentlichungstag der Anmeldung:
21.05.86 Patentblatt 86/21

(84) Benannte Vertragsstaaten:
CH DE FR GB IT LI NL

(71) Anmelder: Howmedica International, Inc.
Zweigniederlassung Kiel
Professor-Küntscher-Strasse 1-5
D-2301 Schönkirchen üb. Kiel(DE)

(72) Erfinder: Hoogland, Thomas, Dr. med.
Akkerveld 1
NL-4854 Bavel(NL)

(72) Erfinder: Harder, Hans Erich
Mecklenburger Strasse 35
D-2316 Probsteierhagen(DE)

(72) Erfinder: Behrens, Klaus, Dipl.-Ing.
Am Sportplatz 8
D-2351 Rickling(DE)

(74) Vertreter: Dipl.-Ing. H. Hauck Dipl.-Phys. W. Schmitz
Dipl.-Ing. E. Graalfs Dipl.-Ing. W. Wehnert Dr.-Ing. W.
Döring
Neuer Wall 41
D-2000 Hamburg 36(DE)

(54) Platte für die Osteosynthese.

(57) Platte für die Osteosynthese, die in Längsrichtung mehrere, im Abstand angeordnete Langlöcher zur Aufnahme von Knochenschrauben aufweist, wobei die Langlöcher (19) nur in einem Längsabschnitt (13) der Platte (10) angeordnet sind, während der andere Längsabschnitt (12) Kreislöcher (17) aufweist und eine auf der Außenseite der Platte anbringbare längliche Schieberplatte (20) vorgesehen ist mit in Längsrichtung beabstandeten Kreislöchern (22), deren Durchmesser annähernd dem kleinen Durchmesser der Langlöcher (19) und deren Teilung der der Langlöcher (19) entspricht.

FIG. 1

32 896-19

PATENTANWÄLTE
DR.-ING. H. NEGENDANK (-1973)

HAUCK, SCHMITZ, GRAALFS, WEHNERT, DÖRING
HAMBURG    MÜNCHEN    DÜSSELDORF

0181433

PATENTANWÄLTE · NEUER WALL 41 · 2000 HAMBURG 36

Howmedica International, Inc.
Zweigniederlassung Kiel
Prof.-Küntscher-Str. 1-5

2301 Schönkirchen üb. Kiel

Dipl.-Phys. W. SCHMITZ · Dipl.-Ing. E. GRAALFS
Neuer Wall 41 · 2000 Hamburg 36
Telefon + Telecopier (040) 36 67 55
Telex 02 11 769 input d

Dipl.-Ing. H. HAUCK · Dipl.-Ing. W. WEHNERT
Mozartstraße 23 · 8000 München 2
Telefon + Telecopier (089) 53 92 36
Telex 05 216 553 pamu d

Dr.-Ing. W. DÖRING
K.-Wilhelm-Ring 41 · 4000 Düsseldorf 11
Telefon (02 11) 57 50 27

ZUSTELLUNGSANSCHRIFT / PLEASE REPLY TO:      HAMBURG, 14. Juni 1984

## Platte für die Osteosynthese

Die Neuerung bezieht sich auf eine Platte für die Osteosynthese,
die in Längsrichtung mehrere, im Abstand angeordnete Langlöcher
zur Aufnahme von Knochenschrauben aufweist.

Die einfachste Osteosyntheseplatte wird von einem länglichen,
flachen, steifen Plattenelement gebildet, das ein oder zwei
Reihen von Kreislöchern aufweist, durch die Knochenschrauben in den
Knochen einschraubbar sind, um die Platte zu beiden Seiten der
Bruchfläche fest mit dem Knochen zu verankern. Eine derartige
Platte hat indessen den Nachteil, daß die Bohrungen im Knochen
vom Chirurgen präzis gesetzt werden müssen, damit eine saubere
Anbringung der Platten erzielt wird. Anderenfalls besteht Gefahr,
daß die Bruchflächen zum Beispiel nicht fest gegeneinander
liegen.

.../2

European Patent Attorneys    Zugelassene Vertreter beim Europäischen Patentamt

Es ist auch bekannt, in Verbindung mit Osteosyntheseplatten
Spannvorrichtungen zu verwenden, um zwischen den Fraktursegmenten
einen ausreichenden Preßdruck aufzubauen, bevor die Platte endgültig fixiert wird.

Es ist bei Osteosyntheseplatten ferner bekannt, Langlöcher
zu verwenden (FR-PS 2 517 536 oder DE-AS 2 806 414). Derartige Langlöcher können an den Enden konisch geformt sein,
so daß mit Hilfe von Knochenschrauben, deren Kopf an der Unterseite konisch oder ballig ausgebildet ist, eine Zugkraft auf
die Platte/ und damit auf die Fraktursegmente aufgebracht werden kann. Derartige Osteosyntheseplatten werden auch als Kompressionsplatten bezeichnet. Mit
Ihnen soll einPreßdruck in der Bruchfläche erzeugt werden.
Nachteilig bei derartigen Platten ist, daß auch hier die Bohrungen vom Chirurgen sehr genau gesetzt werden müssen, um den
ohnehin relativ  kleinen Verstellweg ausnützen zu können.
Wird bezüglich eines Langlochs dieser Verstellweg nur teilweise oder gar nicht erreicht, läßg sich auch bezüglich der
anderen Langlöcher keine Spannung mehr erzeugen.

Während des Heilungsprozesses stellt sich eine sogenannte
Sinterung des Bruches ein, die mit einer Verkürzung des
Knochens in diesem Bereich einhergehen kann. Um sicherzustellen,
daß die Fixierung mit Hilfe der Osteosyntheseplatte  dem
folgen kann, müssen sich die Schrauben in den Langlöchern
relativ zur Platte bewegen. Zwischen Schraubenkopf und Platte findet jedoch im wesentlichen nur Punktkontakt statt. Aufgrund der hohen Flächenpressung an den Kontaktpunkten gräbt sich der Schraubenkopf mehr oder weniger in das Material der Platte ein und verursacht einen außerordentlich hohen Haftreibungswert, der praktisch verhindert, daß bei den normalerweise auftretenden Kräften die beschriebene Relativbewegung erreicht werden kann. Außerdem ist auf diese Weise nicht möglich, reproduzierbar ein gegebenes Drehmoment auf die Knochenschrauben aufzubringen. Die bekannten Osteosyntheseplatten haben zudem über die gesamte Länge Langlöcher. Selbst wenn eine Relativbewegung möglich ist, stellt sich eine Art schwimmende Halterung oder Lagerung der Platte ein und hebt den gewünschten Fixationseffekt letztlich auf.

Der Neuerung liegt daher die Aufgabe zugrunde, eine Platte für die Osteosynthese zu schaffen, die eine stabile Fixation der Fraktursegmente ermöglicht bei gleichzeitiger Anpassung an Relativverschiebungen der Fraktursegmente im wesentlichen parallel zur Osteosyntheseplatte.

Diese Aufgabe wird neuerungsgemäß dadurch gelöst, daß die Langlöcher nur in einem Längsabschnitt der Platte angeordnet sind, während der andere Längsabschnitt Kreislöcher aufweist und eine auf der Außenseite der Platte anbringbare längliche Schieberplatte vorgesehen ist mit in Längsrichtung beabstandeten Kreislöchern, deren Durchmesser annähernd dem kleinen Durchmesser der Langlöcher und deren Teilung der Teilung der Langlöcher entspricht.

Bei der neuerungsgemäßen Osteosyntheseplatte weist nur ein Längsabschnitt Langlöcher auf. Dadurch wird die Platte auf einer Seite des Bruches unverschieblich am Knochen mit Hilfe einer oder mehrerer Knochenschrauben fixiert. Dem Längsabschnitt mit den Langlöchern ist eine Schieberplatte zugeordnet, die an der dem Knochen abgewandten Seite der Platte anliegt und mit Kreislöchern versehen ist, deren Teilung der Teilung der Langlöcher entspricht. Die Knochenschrauben werden durch die Kreislöcher der Schieberplatte und anschließend durch die Langlöcher hindurchgeführt und in den Knochen geschraubt.

Zwischen den Schraubenköpfen und der Schieberplatte einerseits und der Schieberplatte und der Osteosyntheseplatte andererseits werden ausreichende Druckkräfte aufgebracht, so daß bei den normalen Belastungen, wenn die Fraktursegmente gegeneinander anliegen, eine Relativverschiebung zwischen den genannten Teilen nicht auftritt. Würde sich jedoch infolge einer Fraktursinterung ein, wenn auch kleiner Spalt im Bereich der Bruchfläche bilden, ermöglicht die Schieberplatte aufgrund der verhältnismäßig geringen Flächenpressung eine entsprechende Anpassungs-/verschiebung.

Auf diese Weise ist auch bei derartigen Vorgängen in der Fraktur eine wirksame Stabilisierung gegeben. Vor allem wird mit der neuerungsgemäßen Osteosyntheseplatte erreicht, daß die Fraktursegmente mit der natürlichen Flächenpressung belastet werden, die bei Bewegung des Patienten, etwa im Femur oder der Tibia, entstehen. Die neuerungsgemäße Osteosyntheseplatte ermöglicht mithin eine dynamische Bruchbelastung. Eine dynamische Bruchbelastung beschleunigt den Heilungsprozeß merklich und erlaubt dem Patienten den gebrochenen Knochen innerhalb relativ kurzer Zeit wieder in annähernd normalem Ausmaß zu belasten.

In einer weiteren Ausgestaltung der Neuerung ist vorgesehen, daß die Schieberplatte in Querrichtung gewölbt ist und ihre konkave Seite gegen die Platte anliegt. Die Schieberplatte entfaltet bei einer derartigen Ausführung Federwirkung, wenn sie mit Hilfe des Kopfes einer Knochenschrauben gegen die Osteosyntheseplatte angepreßt wird. Auf diese Weise läßt sich eine gewünschte Anpressung relativ präzise einstellen und dadurch auch die relative Druckkraft, die erforderlich ist, um eine Verschiebung der Schieberplatte gegenüber der Osteosyntheseplatte zu erreichen.

Eine weitere Ausgestaltung der Neuerung sieht vor, daß die Platte einen sich in Längsrichtung erstreckenden, zur Außenseite offenen Kanal aufweist, dessen Boden die Langlöcher aufweist und in dem die Schieberplatte geführt ist. Bei Verwendung einer normalen flachen Osteosyntheseplatte würde der Schieber zu einer entsprechenden Volumenvergrößerung des Osteosynthesehilfsmittels führen und die Gefahr von Gewebereizungen verstärken. Die Anbringung der Schieberplatte in einem Kanal verringert die Auftragung durch die Schieberplatte. Gleichwohl wird das Flächenträgheitsmoment der Osteosyntheseplatte nicht nennenswert beeinflußt.

Besonders vorteilhaft ist, wenn in einer weiteren Ausgestaltung der Neuerung die Osteosyntheseplatte im Querschnitt annähernd U-förmig ist. Um ein etwa gleiches Flächenträgheitsmoment zu erzielen wie bei flachen Osteosyntheseplatten, müssen zwar die Schenkel etwas länger gewählt werden als die Dicke der bekannten Platte, dafür liegt jedoch die Schieberplatte, die relativ zur Osteosyntheseplatte eine geringe Dicke aufweist, völlig im Kanal der Platte versenkt. Da auch der Kopf der Knochenschraube versenkt liegt und nur minimal über die Außenseite vorsteht, ist die gesamte Abmessung des Osteosynthesehilfsmittels in Richtung der Knochenschraubenachsen kaum größer oder sogar kleiner als bei herkömmlichen Osteosyntheseplatten. An dieser Stelle sei angemerkt, daß es auch vorteilhaft sein kann, eine derartige neuerungsgemäße Osteosyntheseplatte ohne eine Schieberplatte zu verwenden.

Um die Gefahr von Gewebereizungen zu verringern, sieht eine weitere Ausgestaltung der Neuerung vor, daß die Höhe der Schenkel zu den Enden stetig abnimmt. An den Enden nimmt das erforderliche Biegemoment bekanntlich ab, so daß auch ein geringes Flächenträgheitsmoment in Kauf genommen werden kann. Dementsprechend kann die Höhe der Schenkel reduziert werden.

Um eine gute Anlage an einem Röhrenknochen zu erhalten, sieht eine Ausgestaltung der Neuerung vor, daß die dem Knochen zugeordnete Seite der Platte eine sich in Längsrichtung erstreckende Hohlkehle aufweist.

Die Neuerung wird nachfolgend anhand von Zeichnungen näher
erläutert.

Fig. 1 zeigt eine Druafsicht auf eine schematisch dargestellte
Osteosyntheseplatte nach der Neuerung.

Fig. 2 zeigt einen Schnitt durch die Platte nach Fig. 1
entlang der Linie 2-2.

Fig. 3 zeigt einen Schnitt durch die Platte nach Fig. 1 entlang der Linie 3-3.

Fig. 4 zeigt eine Draufsicht auf eine Schieberplatte für eine
Osteosyntheseplatte nach den Figuren 1 bis 3.

Fig. 5 zeigt einenSchitt durch die Schieberplatte nach Fig. 4
entlang der Linie 5-5.

Bevor auf die  in den Zeichnungen dargestellten Einzelheiten
näher eingegagen wird, sei vorangestellt, daß jedes der beschriebenen Merkmale für sich oder in Verbindung mit Merkmalen
der Ansprüche von neuerungswesentlicher Bedeutung ist.

Die Zeichnungen sind lediglich schemahaft und nicht maßstäblich.

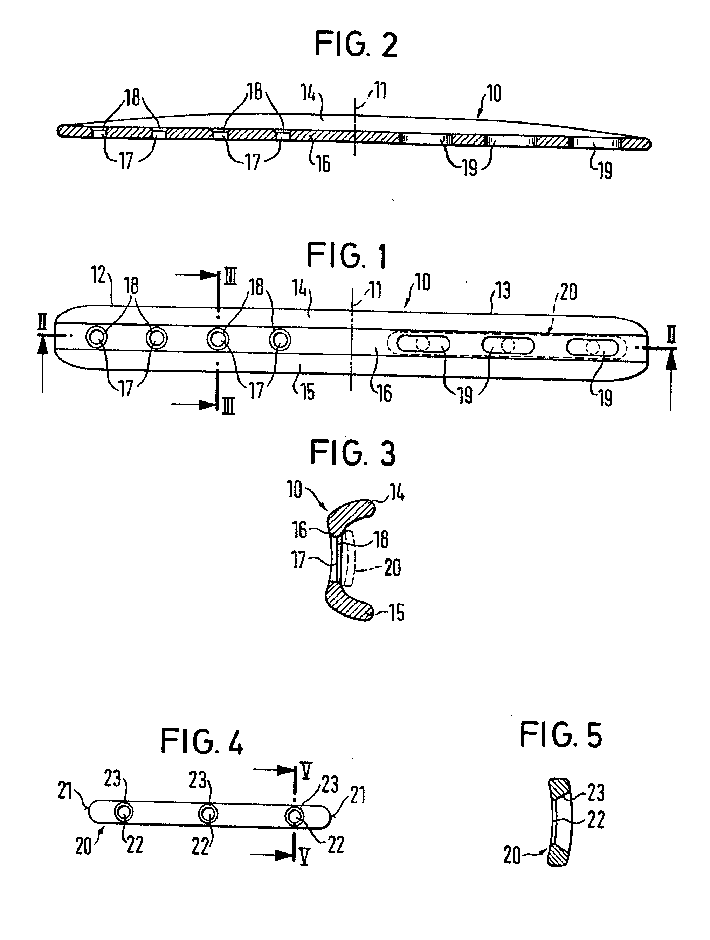

Eine verhältnismäßig schmale längliche Osteosyntheseplatte ist in Figuren 1 bis 3 allgemein mit 10 bezeichnet. Ihre Länge kann z.B. zwischen 200 mm (Femur) und 125 mm (Tibia) schwanken. Entsprechend kann die Breite zwischen 16 und 14 mm betragen. Die Platte 10 ist beidseits einer Mittellinie 11 in einen ersten Längsabschnitt 12 und einen zweiten Längsabschnitt 13 unterteilt. Wie aus der Gesamtbetrachtung der Figuren 1 bis 3 hervorgeht, ist die Platte im Querschnitt U-förmig und weist mithin zwei Schenkel 14, 15 und einen dazwischenliegenden Steg 16 auf. Wie aus Fig. 2 hervorgeht, nimmt die Höhe der Schenkel 14, 15 zu den Enden allmählich ab.

Mit dem Mittelpunkt auf der Längsachse sind im Längsabschnitt 12 vier Kreislöcher 17 im Boden oder Steg 16 geformt. Sie haben eine gleichmäßigen Abstand voneinander und besitzen auf der den Schenkeln 14, 15 zugewandten Seite eine Ansenkung 18. Die Löcher 17 dienen zur Aufnahme an sich bekannter Knochenschrauben, deren Kopf teilweise von der Ansenkung 18 aufgenommen wird. Der andere Längsabschnitt 13 besitzt im gleichmäßigenAbstand angeordnete Langlöcher 19, die beispielsweise eine Länge von 15 mm aufweisen. An den Enden sind die Langlöcher 19 kreisförmig gerundet. Der kleine Durchmesser der Langlöcher 19 ist so bemessen, daß Knochenschrauben durch die Langlöcher 19 hindurchgeführt werden können. Die Langlöcher 19 weisen eine glatte achsparallele Wandung auf ohne Einsenkung.

In den Figuren 4 und 5 ist eine Schieberplatte 20 dargestellt, die aus dem gleichen körperverträglichen Material wie die Platte 10 bestehen kann. Die Schieberplatte 20 ist weitaus dünner als die Platte 10 (die Schnittdarstellungen nach den Figuren 3 und 5 sind vergrößert und geben diese Dickenrelation nicht wieder). Während der Steg oder Boden 16 z.B. eine Dicke von 2,8 bis 3,4 mm aufweisen kann, hat die Schieberplatte 20 eine Dicke von etwa 1,5 mm. Die Schieberplatte 20 ist an den Enden kreisförmig abgerundet, wie bei 21 gezeigt. Wie aus Fig. 5 erkennbar, ist die Schieberplatte 20 in Querrichtung gebogen. Sie besitzt drei im gleichen Abstand zueinander angeordnete Kreislöcher 22 mit einem Durchmesser, der dem kleinen Durchmesser der Langlöcher 19 entspricht. Die Kreislöcher 22 haben eine Ansenkung 23.

Bei der Applikation wird zunächst der Endabschnitt 12 der Osteosyntheseplatte 10 am Knochen fixiert mit Hilfe von nicht gezeigten Knochenschrauben, die durch die Kreislöcher 17 geführt sind. Anschließend wird die Schieberplatte 20 in den durch die Schenkel 14, 15 und den Boden 16 gebildeten Kanal eingelegt, wie gestrichelt in den Figuren 1 und 3 angedeutet. Die Teilung der Löcher 22 der Schieberplatte 20 entspricht der Teilung der Langlöcher 19. Knochenschrauben werden durch die Kreislöcher 22 der Schieberplatte 20 und durch die Langlöcher 19 in den Knochen eingeschraubt, nachdem die Fraktursegmente gerichtet worden sind. Die Knochenschrauben bzw. die Löcher 22 der Platte 20 sind derart angeordnet, daß sie nach Möglichkeit nahe dem in Fig. 1 rechten Ende der Langlöcher 19 liegen. Der Kopf der Knochenschrauben preßt die Schieberplatte fest gegen den Boden 16, wobei sich die Schieberplatte 20 federnd verformt unter teilweiser Aufhebung der Wölbung. Durch ein entsprechendes Drehmoment, z.B. 2,5 Nm an den Knochenschrauben wird eine ausreichende Pressung zwischen den Teilen erhalten, um eine ausreichende Stabilisierung des Bruches zu erzielen. Die maximale Flächenpressung ist jedoch verhältnismäßig gering, so daß sich die Knochenschrauben in den Langlöchern bewegen können, wenn die Fraktursegmente näher aneinander rücken. Eine derartige Relativbewegung der Knochenschrauben ist jedoch mit einer Verschiebung der Schieberplatte 20 gekoppelt. Aufgrund der relativ niedrigen Flächenpressung kann die Schieberplatte unter entsprechender Reibung im Kanal der Platte 10 gleiten.

Es versteht sich, daß alle Kanten der Platte 10 und der Schieberplatte 20 ausreichend verrundet sind, um Gewebereizungen nach Möglichkeit auszuschließen.

Ansprüche

1. Platte für die Osteosynthese, die in Längsrichtung mehrere, im Abstand angeordnete Langlöcher zur Aufnahme von Knochenschrauben aufweist, dadurch gekennzeichnet, daß die Langlöcher (19) nur in einem Längsabschnitt (13) der Platte (10) angeordnet sind, während der andere Längsabschnitt (12) Kreislöcher (17) aufweist und eine auf der Außenseite der Platte anbringbare längliche Schieberplatte (20) vorgesehen ist mit in Längsrichtung beabstandeten Kreislöchern (22), deren Durchmesser annähernd dem kleinen Durchmesser der Langlöcher (19) und deren Teilung der Langlöcher (19) entspricht.

2. Platte nach Anspruch 1, dadurch gekennzeichnet, daß die Schieberplatte (20) in Querrichtung gewölbt ist und ihre konkave Seite gegen die Platte (10) anliegt.

3. Platte nach Anspruch 1 oder 2, dadurch gekennzeichnet, daß die Kreislöcher (22) der Schieberplatte (20) an der Außenseite eine Ansenkung (23) aufweisen.

4. Platte nach einem der Ansprüche 1 bis 3, dadurch gekennzeichnet, daß sie einen sich in Längsrichtung erstreckenden, zur Außenseite offenen Kanal aufweist, dessen Boden (16) die Langlöcher (19) aufweist und in dem die Schieberplatte (20) geführt ist.

5. Platte nach Anspruch 4, dadurch gekennzeichnet, daß sie im Querschnitt annähernd U-förmig ist.

6. Platte nach Anspruch 5, dadurch gekennzeichnet, daß die Höhe der Schenkel zu den Enden stetig abnimmt.

7. Platte nach einem der Ansprüche 4 bis 6, dadurch gekennzeichnet, daß die dem Knochen zugewandte Seite der Platte (10) eine sich in Längsrichtung erstreckende Hohlkehle (18) aufweist.

1/1

0181433

FIG. 2

FIG. 1

FIG. 3

FIG. 4

FIG. 5

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | US-A-3 659 595  (E.J. HABOUSH) <br><br> * Spalte 2, Zeilen 56-68; Abbildungen 1,7-9 * | 1,3-5, 7 | A 61 B  17/58 |
| Y | | 2,6 | |
| Y | FR-A-1 239 266  (H.-G. VAN STEENBRUGGHE) <br> * Abbildungen 3,8 * | 2,6 | |
| A | | 1,3-5, 7 | |
| X | CH-A-  373 516  (M.E. MÜLLER) <br> * Seite 3, Zeilen 1-32; Abbildungen 3-5 * | 1,3,4 | |
| X | US-A-2 406 832  (M.G. HARDINGE) <br><br> * Insgesamt * | 1,4,5, 7 | **RECHERCHIERTE SACHGEBIETE (Int Cl 4)** <br><br> A 61 B |
| X | DE-C-  867 422  (A. ABEL) <br> * Abbildungen 1,2 * | 1 | |
| A | EP-A-0 013 862  (OSTEO) <br> * Abbildungen 1-3 * | 1-4,7 | |
| | ---          -/- | | |

Der vorliegende Recherchenbericht wurde für alle Patentansprüche erstellt

| Recherchenort <br> DEN HAAG | Abschlußdatum der Recherche <br> 31-01-1986 | Prüfer <br> WOLF C.H.S. |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

# EUROPÄISCHER RECHERCHENBERICHT

**0181433**
Nummer der Anmeldung

EP 85 10 7410

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | US-A-3 695 259 (C.E. YOST) <br> * Insgesamt * <br><br> --- | 2-5,7 | |
| A | FR-A-2 405 061 (R. DAYAN) <br> * Seite 1, Zeilen 22-25; Seite 2, Zeilen 36-40; Abbildungen 1-9 * <br><br> ----- | 2,4-7 | |

RECHERCHIERTE SACHGEBIETE (Int Cl 4)

Der vorliegende Recherchenbericht wurde für alle Patentansprüche erstellt

| Recherchenort <br> DEN HAAG | Abschlußdatum der Recherche <br> 31-01-1986 | Prüfer <br> WOLF C.H.S. |
|---|---|---|